# EUROPEAN PATENT SPECIFICATION

(11) **EP 2 246 337 B1**
(45) Date of publication and mention of the grant of the patent: **21.12.2011**
(21) Application number: 10005150.7
(22) Date of filing: 12.01.2005
(51) Int. Cl.: C07D 261/08, C07D 413/04, A61K 31/42

(54) **Isoxazole derivatives for use as cyclooxygenase inhibitors**
Isoxazol-Derivate zur Anwendung als Cyclooxygenase-Inhibitoren
Dérivés d'isoxazole pour leur utilisation comme inhibiteurs de la cyclooxygénase

(30) Priority: 12.01.2004 IT MI20040019
(43) Date of publication of application: 03.11.2010
(62) Divisional of application: 05702217.0
(73) Proprietor: Universita' degli Studi di Bari, 70121 Bari (IT); Universita' Degli Studi di Chieti, 66013 Chieti (IT)
(72) Inventor: Scilimati, Antonio, 70125 Bari (IT); Di Nunno, Leonardo, 70125 Bari (IT); Vitale, Paola, 70125 Bari (IT); Patrignani, Paola, 66013 Chieti (IT); Tacconelli, Stefania, 66013 Chieti (IT); Porreca, Ettore, 66013 Chieti (IT); Stuppia, Liborio, 66013 Chieti (IT)
(74) Representative: Trupiano, Federica

(56) References cited:
- EP-A- 0 026 928
- WO-A-03/029230
- ROSTON, D. A. ET AL: "Comparison of drug substance impurity profiles generated with extended length columns during packed-column SFC" JOURNAL OF PHARMACEUTICAL AND BIOMEDICAL ANALYSIS, vol. 26, no. 3, 2001, pages 339-355, XP002335573
- DATABASE CA [Online] CHEMICAL ABSTRACTS SERVICE, COLUMBUS, OHIO, US; 1980, BELLEC, CHRISTIAN ET AL: "Preparation of trisubstituted isoxazoles by chemical and electrochemical reduction of .alpha.-acyl-.beta.-nitrostilbenes" XP002335581 retrieved from STN Database accession no. 1980:426329 & JOURNAL OF HETEROCYCLIC CHEMISTRY , 16(8), 1657-9 CODEN: JHTCAD; ISSN: 0022-152X, 1979,

## Description

### BACKGROUND OF THE ART

The term "Non Steroidal Anti-Inflammatory Drugs" (NSAID) refers to a class of drugs that has been known for a long time and which is able to reduce inflammation, pain and fever. The term "non steroidal" distinguishes these drugs from corticosteroids with an anti-inflammatory activity, which are agents with a more marked anti-inflammatory activity, but which present significant and important side effects. The most known and used drugs belonging to the class of non-steroidal anti-inflammatory drugs (NSAID) are, for example, acetylsalicylic acid (*Aspirin*®) and the corresponding salicylates, ibuprophene (currently available on the market as *Advil*® and *Motrin*®), naproxene (currently available on the market as *Naprelan*® and *Alive*®) and indomethacin (currently available on the market as *Indocin*®).

It has been ascertained that these drugs act by means of an action mechanism which blocks the synthesis of prostaglandin through the inhibition of the enzyme Cyclooxygenase (COX), known also as Prostaglandin G/H Synthase (PGHS). Through this action mechanism, the drugs belonging to the class of non-steroidal anti-inflammatory drugs (NSAID) show, alongside the anti-inflammatory action, negative side effects on the gastrointestinal apparatus, because prostaglandin synthesis is involved in both processes.

For many years it was therefore believed that it was impossible to obtain non-steroidal anti-inflammatory drugs (NSAID) that were not characterised by significant side effects on the gastrointestinal system. Later, after having shown that the enzyme cyclooxygenase (COX), which catalyses the conversion of arachidonic acid to prostaglandin and thromboxane, exists in two isoforms, known as COX-1 and COX-2, which proved to be codified by two different genes, it was also ascertained that the form indicated as cyclooxygenase-1 (COX-1) is virtually expressed in a constitutive way in all tissues and is therefore indicated as "constitutive", while the form cyclooxygenase-2 (COX-2) is expressed in response to stimuli, for example of an inflammatory nature, and is indicated as "induced", even though many exceptions are known to the generalisation given above.

In particular, the "constitutive" form COX-1 is involved in the activity of the digestive tract, while the "induced" COX-2 isoform is involved in the inflammatory process.

Therefore it clearly appears the necessity to develop new drugs which selectively inhibit the "induced" form COX-2, involved in the inflammatory process, and which drugs are not inhibitors, or at least not selective inhibitors, of only the "constitutive" form COX-1, thus avoiding even serious adverse side effects on the gastrointestinal system.

Selective COX-2 inhibitors therefore possess a significant pharmacological activity as non-steroidal anti-inflammatory drugs for the treatment of acute pain syndromes and chronic disorders of an inflammatory nature. For example, selective COX-2 inhibitor such as Rofecoxib, Celecoxib (currently available on the market with the name Celebrex®) and Valdecoxib (currently available on the market with the name Bextra®) are advantageously used for the treatment of rheumatoid arthritis, osteoarthritis, and for the treatment of acute pain associated, for example, with dental surgery and with primary dysmenorrhoea. Recently, Rofecoxib, trade name Vioxx®, was withdrawn from the market due to data deriving from a study carried out on patients treated, who showed an alarming incidence of myocardial infarct and ischemia in comparison with the non treated group. In fact, this product, while demonstrating a good anti-inflammatory activity and the absence of side effects on the gastrointestinal system, showed, on the basis of these initial studies, serious and significant side effects on the cardiovascular system. For this reason several studies have been undertaken to assess any side effects on the cardiovascular system of selective anti-inflammatory product COX-2 inhibitors.

It therefore appears clear that the use of derivatives known as COX-2 inhibitors involves a series of consistent and dangerous side effects in the cardiovascular field, with respect to what has been shown for the non-steroidal anti-inflammatory drugs that have been in use since a long time. On the other hand, these compounds remain highly efficacious drugs for the treatment of inflammatory states, especially of medium and severe extent, and it is therefore important to interpret correctly the most significant side effects on the cardiovascular system that have recently been revealed.

Also during studies of cyclooxygenase inhibitors, a significant role of the constitutive form COX-1 was demonstrated in the development of carcinogenic pathologies, in particular in the development of intestinal polyposis and in the onset of cutaneous and ovarian carcinomas. In parallel, it was also observed that COX-1 plays an important role in the onset, for example following surgery, of medium and strong pain. Moreover, it was seen that the administration of COX-1 inhibitors can be useful in the prevention and/or treatment of atherosclerosis. It is therefore important to select and identify specific cyclooxygenase inhibitors even of the "constitutive" COX-1 form.

### OBJECTS OF THE INVENTION

The object of the present invention is to provide the use of two compounds inhibitors of cyclooxygenase (COX-1 and/or COX-2), in particular 3,4-diaryl isoxazole derivatives, which show a pharmacological activity and which are therefore used in the treatment of inflammation, in the prevention and treatment of carcinomas, in particular intestinal, ovarian and cutaneous, and in the treatment of pain syndromes, in particular resulting from surgery.

Yet another object of the present invention is to provide a pharmaceutical composition which comprises one of the above cyclooxygenase inhibitor (COX-1 and/or COX-2), which is effectively used in the treatment of inflammation, in the prevention and treatment of carcinomas, in particular intestinal, ovarian and cutaneous, and in the treatment of pain, in particular resulting from surgery.

### DESCRIPTION OF THE INVENTION

These and yet other objects and respective advantages which will be better explained by the following description, are achieved by cyclooxygenase inhibitor compounds (cox-1 and/or COX-2), selected from:
- compound P10 which is 3,4-diphenyl-5-methylisoxazole; and
- compound P9 which is 3,4-diphenyl-5-ethylisoxazole.

The above 3,4 diaryl isoxazole derivatives, are generally prepared by reaction between arylnitryle oxides and free enolate ions (in turn obtained by metallation reaction of various alkyl methyl ketones with LDA in suitable conditions), followed by a dehydration/aromatization reaction and if necessary by further reactions of derivatization/modification of the derivatives thus obtained.

The synthetic procedure for the preparation of the above 3,4-diaryl isoxazole derivatives is disclosed in the following examples.

### EXAMPLE 1

### Synthesis of the product P9 (3,4-diphenyl-5-ethylisoxazole),

*n*-Butyllithium in hexane (2.19M, 0.213 mL, 0.4675 mmol) was added to 3,4-diphenyl-5-methylisoxazole (0.100 g, 0.425 mmol) in THF (5 mL) kept under stirring at -78 °C under nitrogen, using a three-neck flask under a flow of nitrogen with magnetic stirring, an entry for nitrogen and a dropping funnel. The red reaction mixture obtained was kept under stirring for 1 hour at -78 °C before adding CH₃I (4.25 mmol). The reaction mixture was brought to room temperature and then processed by adding NH₄Cl aqueous solution. The two phases were separated and the aqueous phase was extracted three times with ethyl acetate. The combined organic extracts were dried on anhydrous Na₂SO₄ and then the solvent was evaporated in a vacuum. The residue was subjected to column chromatography (silica gel, petroleum ether: ethyl acetate = 10/1) and 3,4-diphenyl-5-ethylisoxazole was obtained with a yield of 75 %.
**3,4-Diphenyl-5-ethyllsoxazole (2g)**. 75% yield. mp 85-87°C (hexane), white crystals. FT-IR (KBr): 3029, 3005, 2923, 2848, 1625, 1596, 1493, 1467, 1437, 1410, 1327, 1282, 1210, 1011, 905, 771, 702 cm⁻¹. ¹H NMR (200 MHz, CDCl₃ δ): 1.29 (t, 3H), 2.78 (q, 2H); 7.12-7.43 (m, 10H). ¹³C NMR (75 MHz, CDCl₃ δ): 12.52, 19.67, 115.20, 127.90, 128.67, 128.93, 129.41, 129.53, 130.16, 130.66, 161.34, 171.43. GC-MS (70 eV) *m*/*z* (rei.int.): 249 (M⁺, 100), 234 (6), 221 (18), 220 (99), 194 (9), 193 (61), 192 (46), 165 (17), 115 (7), 103 (8), 89 (53), 77 (15), 63 (10), 51 (10). Anal. calc for C₁₇H₁₅NO: C, 81.90: H, 6.06; N, 5.62. Found: C, 81.92; H, 6.02; N, 5.60.

### EXAMPLE 2

Analytical data of the product P10 (2a) **3,4-Diphanyl-5-methylisoxazole (2a)**.
80% yield. mp 97-98°C (hexane), white crystals. FT-IR (KBr): 3051, 2928, 1619, 1597, 1573, 1497, 1464, 1436, 1414, 1376, 1304, 1239, 1074, 915, 769, 696 cm⁻¹, ¹H NMR (CDCl₃, δ): 2.45 (s, 3H); 7.17-7.47 (m, 10H) ¹³C NMR (75 MHz, COCl₃, δ): 11.82, 116.01, 127.90, 128.69, 128.95, 129.38, 129,60, 130.05, 130.61, 161.39, 166.85. GC-MS (70 eV) *m*/*z* (rel.int.): 235 (M⁺, 100), 220 (28), 194 (14), 193 (90), 192 (37), 165 (28), 103 (10), 90 (12), 89 (62), 78 (10), 77 (24), 63 (23), 51 (48), 43 (70). Anal. calculated for C₁₆H₁₃NO: C, 81.68; H, 5.57; N, 5.95. found: 81.73; H, 5.50; N, 5.95.

The products obtained according to the present invention were tested with an in vitro test on whole human blood isolated as described in Patrignani P. et al., J Pharmacol Exp Ther 1994; 271:1705-12*;* Patrignani P. et al., J Clin Invest 1982;69:366-1372*;* Patrono C. et al.. Low dose aspirin and inhibition of thromboxane B2 production in healthy subjects. Thromb. Res. 1980; 17: 317-27. The assays were carried-out to assess the-effects of the substituents and the activity of the compounds according to the present invention as inhibitors of COX-1/COX-2 activity, taking Valdecoxib (present trade name *Bexfra*®) as a reference.

11 healthy volunteers (6 females aged between 25 and 29 years) were enrolled to participate in the study after each having given their written approval. The same volunteers had previously taken part in other studies.

### COX-2 Assay

1 ml aliquots of a sample of isolated peripheral venous blood samples containing 10 IU of sodium heparin were incubated in the laboratory in the presence of LPS (lipopolysaccharide) (10microg/ml) or saline for 24 hours at 37°C as described. The contribution of platelet COX-1 activity was suppressed by pre-treating the subjects with 300 mg of aspirin 48 hours before blood sampling. Plasma was separated by centrifugation (10 minutes at 2.000 r.p.m.) and kept at -70°C until assayed for prostaglandin (PG)E₂, as an index of LPS-induced monocyte COX-2 activity.

### COX-1 Assay.

Samples of isolated peripheral venous blood were used in vitro (taken from the same donors when they had not taken any non-steroidal anti-inflammatory drug during the two weeks preceding the study). 1 ml aliquots of isolated whole blood were immediately transferred into glass test tubes and kept at a temperature of 37°C for 1 hour. The serum was separated by centrifugation (10 min at 3000 rpm) and kept at -70°C until assayed for thromboxane (TX)B₂. (TX)B₂ whole blood production was measured as a reflexion of the maximally stimulated platelet COX-1 activity in response to endogenous formed thrombin.

### Effect of the tested compounds on isolated whole blood COX-1 and COX-2 activities.

The compounds (0.005-150 mM) were dissolved in DMSO and aliquots of 2 microlitres of the solutions were pipetted directly into test tubes to give final concentrations of 0.01-300 microM in the blood. From 4 to 9 different concentrations of each compound were incubated with heparinised isolated whole blood samples in the presence of LPS (10 micrograms/ml) for 24 hours or with isolated whole blood samples allowed to clot at 37°C for 1 hour, in order to examine the concentration-dependence of COX-2 vs. COX-1 inhibition, respectively.

### Analysis of PGE₂-and TXB₂

The concentrations of PGE₂ and TXB₂ were measured by radioimmunological assays (Patrono C. et al., Low dose aspirin and inhibition of thromboxane B2 production in healthy subjects. Thromb. Res. 1980; 17: 317-27; Ciabattoni G. et al. J Endocrinol Invest 1979; 2:173-182). Isolated plasma and serum samples were diluted in the standard diluent of the assay (0.02M phosphate buffer, pH 7.4) and assayed in a volume of 1.5 ml at a final dilution of 1:50-1:30.000. 4000 d.p.m. of [³H]-PGE₂ or [³H]-TXB₂ were used and specific anti-PGE₂ and anti-TXB₂ antibody diluted 1:100,000 and 1:120,000, respectively. The least detectable concentration was 1-2 pg/ml for both prostanoids.

### Results

LPS-stimulated isolated whole blood samples, drawn from healthy subjects treated with aspirin 300 mg 48 h before sampling, produced 33±5.8 ng of PGE₂ per ml of plasma (mean±S.E.M., n=13). TXB₂ production in clotting isolated whole blood samples, obtained from the same subjects in aspirin-free periods, averaged 466±53 ng/ml (mean±S.E.M., n=13).

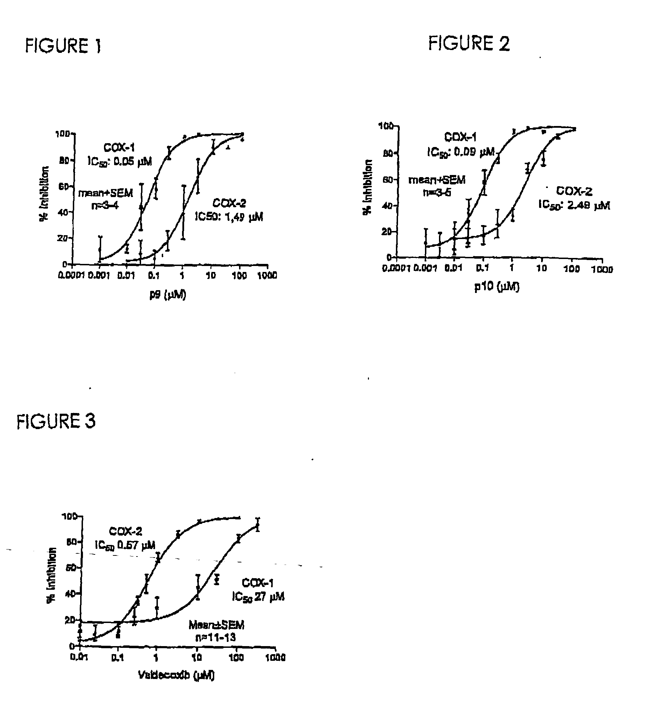

As shown in figures 1, 2, and 3, the compounds P9. P10 and the reference Valdecoxib inhibited LPS-induced monocyte COX-2 and thrombin-stimulated platelet COX-1 activities in a concentration-dependent fashion. IC₅₀ values for inhibition of platelet COX-1 and monocyte COX-2 activities are given in **Table 1**.

Valdecoxib inhibits monocyte COX-2 and platelet COX-1 activities with the following values of IC₅₀: 27 (16.22-44.63) and 0.57 (0.4619-0.7056) microM (95% interval).

The analysis of the sigmoidal concentration-response curves for inhibition of monocyte COX-2 and platelet COX-1, showed that virtually complete suppression (>90%) of platelet COX-1 activity occurs at concentrations that do not affect monocyte COX-2 activity with the compounds P9 and P10 (Figure 1 e 2).

**Table 1. Pharmacological data.**

| Compound | COX-1 | COX-2 |
|---|---|---|
| | IC₅₀(µm) | IC₅₀(µM) |
| **2a(P10)** | 0.090^{a} | 2.49^{b} |
| | (0.05-0,144) | (1.577-3.954) |
| | | |
| **2g (P9)** | 0.05^{a} | 1.49^{c} |
| | (0.028-0.097) | (0.812-2.768) |
| | | |
| **Valdecoxib** | CH₃ 27^{d} | 0.57^{e} |
| | (16.22-44.63) | (0.4619-0.7056) |

| | | |
|---|---|---|
| The values are means of at least two experiments ^{a} n=3 ^{b} n=5 ^{c} n=4 ^{d} n=11 ^{e} n=13 (values in parentheses are the IC₅₀ confidence intervals) | | |

## Claims

1. A compound selected from 3,4-diphenyl-5-ethylisoxazole (**P9**) and 3,4-diphenyl-5-methylisoxazole (P10) for use in the inhibition of COX-1/COX-2 for the treatment of inflammatory syndromes, in the prevention and treatment of carcinomas, in particular intestinal, ovarian and cutaneous, and in the treatment of pains, in particular deriving from surgery and In the prevention and/or treatment of atherosclerosis.

2. The compound according to claim 1 for use in the treatment of inflammatory syndromes, in the prevention and treatment of carcinomas, in particular intestinal, ovarian and cutaneous, and in the treatment of pains, in particular deriving from surgery.

3. The compound according to claim 1 for use in the prevention and/or treatment of atherosclerosis.

4. A pharmaceutical composition comprising 3,4-diphenyl-5-ethylisoxazole (**P9**).

5. A pharmaceutical composition comprising 3,4-diphenyl-5-methylisoxazole (**P10**).

## Patentansprüche

1. Eine Verbindung ausgewählt aus 3,4-Diphenyl-5-ethylisoxazol (P9) und 3,4-Dipheny-5-methylisoxazol (P10) zur Verwendung für die Hemmung von COX-1/COX-2 in der Behandlung von entzündlichen Syndromen, in der Vorbeugung und Behandlung von Karzinomen, insbesondere intestinale, Eierstock- und kutane Karzinome und in der Behandlung von Schmerz, insbesondere Operationsschmerz und in der Vorbeugung und/oder Behandlung von Atherosklerose.

2. Die Verbindung gemäß Anspruch 1 zur Verwendung in der Behandlung von entzündlichen Syndromen, in der Vorbeugung und Behandlung von Karzinomen, insbesondere intestinale, Eierstock- und kutane Karzinome und in der Behandlung von Schmerz, insbesondere Operationsschmerz.

3. Die Verbindung gemäß Anspruch 1 zur Verwendung in der Vorbeugung und/oder Behandlung von Atherosklerose.

4. Eine pharmazeutische Zusammensetzung, enthaltend 3,4-Diphenyl-5-ethylisoxazol (P9).

5. Eine pharmazeutische Zusammensetzung, enthaltend 3,4-Dipheny-5-methylisoxazol (P10).

## Revendications

1. Composé choisi à partir de 3,4-diphényl-5-éthylisoxazole (P9) et de 3,4-diphényl-5-méthylisoxazole (P10) à utiliser dans l'inhibition de COX-1/COX-2 pour le traitement de syndromes inflammatoires, dans la prévention et le traitement des carcinomes, en particulier intestinal, ovarien et cutané, et dans le traitement des douleurs, en particulier, provenant d'une intervention chirurgicale et dans la prévention et/ou le traitement de l'athérosclérose.

2. Composé selon la revendication 1, à utiliser dans le traitement de syndromes inflammatoires, dans la prévention et le traitement de carcinomes, en particulier intestinal, ovarien et cutané, et dans le traitement de douleurs, en particulier provenant d'une intervention chirurgicale.

3. Composé selon la revendication 1, à utiliser dans la prévention et/ou le traitement de l'athérosclérose.

4. Composition pharmaceutique comprenant du 3,4-diphényl-5-éthylisoxazole (P9).

5. Composition pharmaceutique comprenant du 3,4-diphényl-5-méthylisoxazole (P10).
